# EUROPEAN PATENT SPECIFICATION

(11) **EP 0 600 921 B1**
(45) Date of publication and mention of the grant of the patent: **27.05.1998**
(21) Application number: 92915954.9
(22) Date of filing: 20.03.1992
(51) Int. Cl.: A61M 5/178, A61B 17/34

(54) **TROCAR WITH RETRACTING TIP**
TROKAR MIT ZURÜCKZIEHBARER SPITZE
TROCART A POINTE RETRACTABLE

(30) Priority: 30.08.1991 US 752838
(43) Date of publication of application: 15.06.1994
(73) Proprietor: ORIGIN MEDSYSTEMS, INC., Menlo Park, CA 94025 (US)
(72) Inventor: MOLL, Frederic, H., San Francisco, CA 94118 (US); COSTA, Peter, F., Winthrop, MA 02152 (US); HOLMES, William, A., Marblehead, MA 01945 (US)
(74) Representative: Weydert, Robert
(86) International application number: US9202261
(87) International publication number: WO9304715

(56) References cited:
- EP-A- 0 479 130
- EP-A- 0 495 633
- EP-A- 0 499 457
- US-A- 4 535 773
- US-A- 4 747 831
- US-A- 4 902 280
- US-A- 4 931 042
- US-A- 4 943 280
- US-A- 5 053 016
- US-A- 5 116 353

## Description

### 1. Field of the Invention

This invention relates to surgical instruments, and specifically to an improved trocar for providing communication with a body cavity.

### 2. Background of the Invention

Trocars are surgical instruments that have a sharp pointed stylet for piercing a body wall to provide an opening for a cannula or tube which forms a part of the trocar and closely surrounds the stylet. After removal of the stylet from its obturating position within the trocar tube, various instruments may be inserted into the cavity through the tube. One such application is to insert an endoscopic instrument in order to perform endoscopic surgery within the cavity.

A common problem with early trocars was the danger associated with an unprotected piercing tip of the stylet. Being very sharp, the point can cause injury to the surgeon if unprotected prior to use. During use, the stylet and trocar tube are forcibly thrust through a body wall until the tube is within the body cavity, so as to provide access thereto from the body exterior. After performing its function of piercing the body wall to admit the tube, the sharp point may cause damage to body tissue and organs within the cavity.

In order to solve these and other problems, trocars were developed that have spring-loaded tubular shields that normally protect and cover the stylet point until the moment that the trocar tube is thrust through the body wall tissue. One such prior art trocar is shown in US-A-4,601,710. An improved version of this prior art device is shown in US-A-4,654,030, on which the two-part form of independent claim 1 is based. With those devices, the surrounding trocar tube is forced back against a biasing spring by the action of passing through the body wall tissue so as to expose the stylet point, which is normally recessed just inside the open distal end of the tube. Once through the body wall, the tube returns to its normal position covering and protecting the point. A locking mechanism is also provided so that the tube may not be accidentally retracted.

EP-A-0 479 130, which has been published after the priority date of the present European patent but is comprised in the state of the art according to Art. 54 (3) EPC, concerns a trocar wherein the sharp pointed stylet is moved from a protected position within the trocar tube to an operative position in advance of the distal end of the tube just before the body wall tissue is pierced. After piercing the tissue and passing into the body cavity, a mechanism in the trocar assembly reacts to a decrease in pressure on the point, and the stylet is quickly and automatically withdrawn to its initial retracted position within the protective trocar tube.

### Summary of the Invention

In accordance with the invention, there is provided a trocar assembly comprising an elongated trocar obturator having a piercing tip at its distal end; a trocar body, said trocar obturator having its proximal end mounted in said body; and an elongated trocar tube mounted on a trocar tube body, said trocar tube body being engageable with said trocar body wherein said obturator is located within said trocar tube, said trocar tube having an open distal end, and a proximal end fixed to said tube body; characterized in that said trocar obturator has its proximal end reciprocably mounted in said trocar body and said trocar assembly having means for extending and retracting said obturator relative to said tube from a first position where said tip is recessed from said distal tube end to a second operative position extended from said distal tube end and thence automatically back to said first position, the latter in response to a distal movement of said tip a predetermined distance incident to a change in force against said tip after the tip passes through a cavity wall, said means for extending and retracting including lever means located in a cavity of said trocar body and operatively connected to said obturator, spring means located in said cavity, said spring means having first spring biasing means for urging said lever means to move said obturator in a proximal direction, and second spring biasing means for urging said lever means to move said obturator in a distal direction, said second spring biasing means effecting said distal movement of said obturator and tip responsive to said change in force acting against said tip and the first spring biasing means being then effective to retract said obturator to said first position.

Advantageous embodiments of the trocar assembly are defined in the dependent claims 2 to 22.

### Brief Description of the Drawings

Fig. 1 is an exploded top quarter isomeric view of the inventive trocar showing its two subassemblies;
Fig. 2A is a top plan cross-sectional view of the inventive trocar in its initial protected position;
Fig. 2B is a similar view showing the trocar advanced from its protective tube;
Fig. 2C is a further view of the same showing the stylet locked into position just prior to piercing a body wall;
Fig. 2D is a still further view of the same showing the stylet beginning to retract after passing through the body wall;
Fig. 3 a top quarter isometric view of the interior of the top half of the trocar head or grip showing details thereof;
Fig. 4A is an exploded top quarter isometric view of the trocar body subassembly;
Fig. 4B is an exploded top quarter isometric view of the trocar tube subassembly; and
Fig. 5 is a top plan cross-sectional view of an alternate embodiment having structure for reducing drag on the stylet.

### Detailed Description

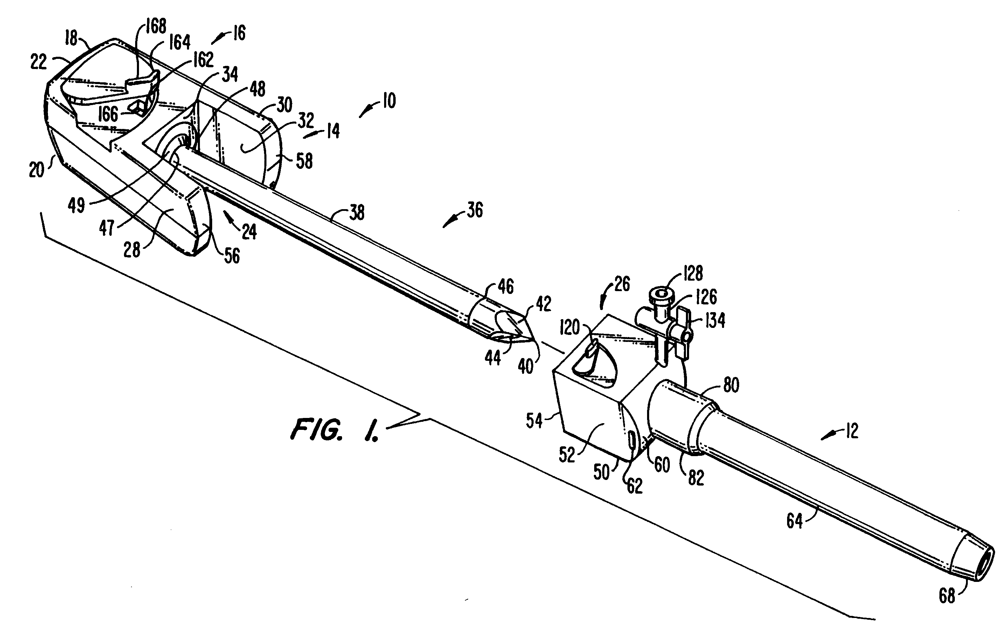

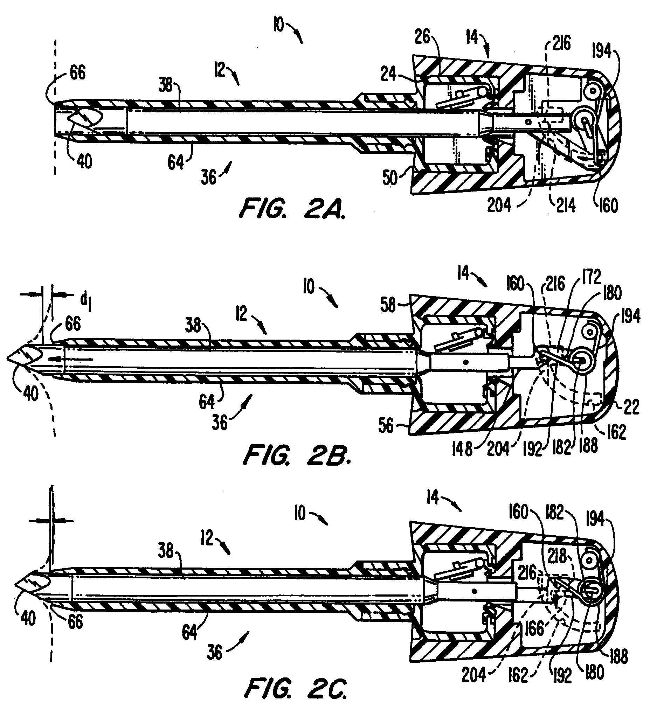

As shown in Fig. 1, the trocar assembly 10 consists of two subassemblies: a trocar tube subassembly 12 and a trocar body subassembly 14. The two subassemblies are designed to be separable from each other. Trocar body subassembly 14 includes a head or grip 16 made up of top and bottom halves 18 and 20, respectively. These top and bottom halves may be made of plastic material such as ABS plastic. The head 16 is generally rectangular with a rounded rear wall 22 adapted to fit the palm of the hand of the surgeon. The front of the head has a rectangular slot 24 therein which is dimensioned to closely receive correspondingly shaped trocar tube body 26, as best seen in Figs. 2A-2D.

Returning to Fig. 1, the slot 24 in head 16 defines a pair of spaced, parallel arms 28, 30 having a pair of opposing side walls, one of which is shown at 32. Projecting between the arms centrally and disposed from front wall 34 is a stylet 36. Stylet 36 is comprised of an elongated obturator 38, which may conveniently be made of aluminum or other material. Stylet 36 has a sharp piercing tip or point 40 which may conveniently be made of aluminum or stainless steel material fixedly mounted on the distal end thereof. The piercing tip is formed by the intersection of three angled surfaces, two of which are shown at 42, 44. The obturator has a tapered surface which narrows from its distal end 46 to its proximal end 48, where it enters aperture 47 in frustoconical projection 49 on the front wall 34 in order to reduce friction and thereby facilitate its movement through body tissue.

Trocar tube subassembly 12 has a body 50, which may be made of plastic material such as ABS plastic, which is dimensioned to closely fit within slot 24. In this position the side walls of the body 50, one of which is shown at 52, will closely contact the accommodating side walls of the head 16. At the same time, a rear wall 54 of the trocar tube body 50 will closely contact front wall 34 of head 16. When in this position, the rounded front walls 56, 58 of head 16 will be in register with a correspondingly shaped rounded front wall 60 of trocar tube body 50. In this manner, the surgeon can conveniently grip the head 16 by placing a finger on each front wall 56, 58, while holding the rounded rear wall 22 in the palm of the hand.

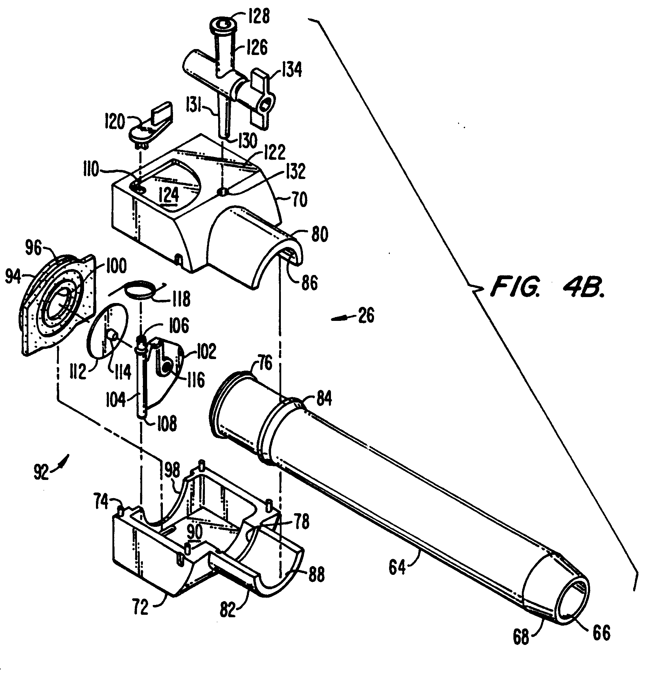

In order to hold the two subassemblies 12, 14 together, projections (one of which is shown at 62), are molded into the side walls of trocar tube subassembly 12. Projecting from the front wall 60 of the tube body 26 is an elongated trocar tube or cannula 64, which may be made of plastic material such as ABS plastic, having a distal end 66 which has a tapered frustoconical surface 68. As best seen in Fig. 4B, the principal elements of the trocar tube subassembly 12 are: the body 26, which is made up of generally symmetrical upper and lower halves 70, 72 which are held together by a plurality of pin projections 74 in lower half 72, which mate with corresponding bores (not shown) in top half 70; and the trocar tube 64 having a circumferential ring 76 formed thereon adjacent its proximal end which fits within annular groove 78 in mating semicircular projections 80, 82. Another ring 84, which is spaced from ring 76 and formed on said trocar tube 64 intermediate ring 76 and the distal end 66 of tube 64, functions to seal off the semicircular openings 86, 88 in projections 80, 82, respectively. While pin projections have been shown, it is to be understood that other fastening means such as screws or adhesive could alternatively be used.

Within chamber 90 formed by the body halves 70, 72 is a flap valve mechanism 92. The flap valve mechanism includes: a generally circular grommet-like seal 94 of deformable material such as rubber, having a groove 96 therearound for mounting in a pair of semicircular holes, one of which is shown at 98 in lower half 72 and having central opening 100 therethrough; a generally U-shaped valve 102 mounted along one edge thereof to a vertically oriented shaft 104, the shaft being pivotally mounted at its upper and lower ends 106, 108 in holes in the tube body, one of which is shown at 110; a circular pad 112, which may be made of a plastic material such as Tygon plastic, fixed to the valve 102 by means of an integral projection 114 which engages bore 116 through the center of valve 102; and a spring 118 which fits over shaft 104. The spring 118 biases valve 102 and thereby pad 112 to normally close off opening 100 when the stylet (not shown) is removed. The function of the flap valve mechanism is to act as a closure when the trocar tube subassembly is removed from the trocar body subassembly.

Parenthetically, grommet-like seal 94 is adapted to seal with the frustoconical projection 49 on the front wall 34 of head 16 (see Fig. 1). Also a part of the flap valve mechanism is an actuating lever 120 which is mounted on the upper end 106 of the shaft exteriorly of the body 72. The exterior top surface 122 of the body has a fan-shaped recess 124 molded therein within which the lever 120 is free to move. The lever 120 allows the valve to be manually opened for admission of surgical instruments. Completing the tube assembly is a valve or stopcock 126 having a valve inlet 128 and a valve outlet 130. A tapered frustoconical portion 131 of the valve 126 is inserted into a bore 132 in top half 70. The valve is actuated by means of rotating a valve handle 134 so as to selectively open or close off communication between inlet 128 and outlet 130, thereby facilitating the admission of insufflating gas to the body cavity.

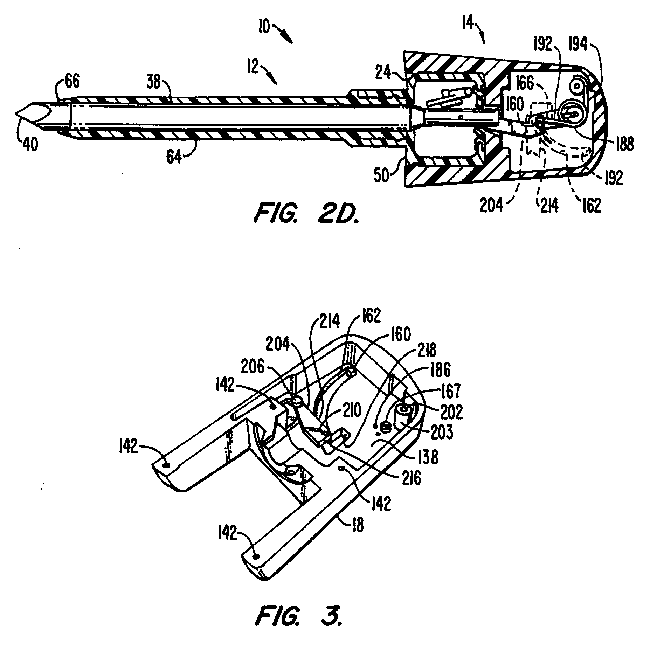

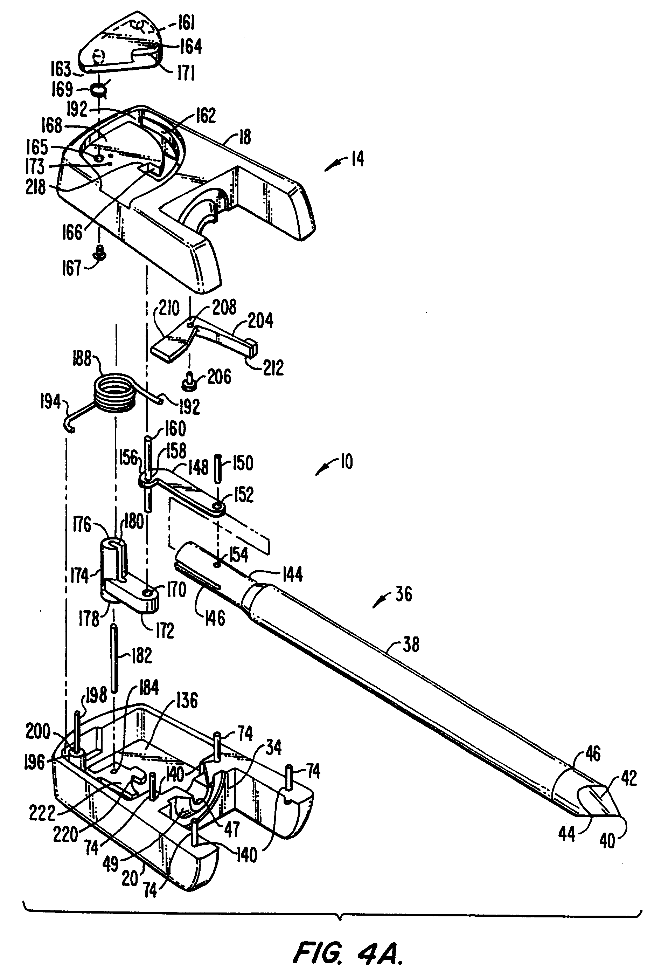

Turning now to Fig. 4A, the top and bottom halves 18, 20 are exploded to show details of the interior mechanism of trocar body subassembly 14. As may be seen, a cavity 136 is formed in the bottom half 20. As best seen in Fig. 3, a mating cavity 138 is formed in top half 18. Returning to Fig. 4A, the top and bottom halves are joined together by means of a plurality of metal pins 74 or other convenient fastening means. As may be seen, these pins are press fit into accommodating bores 140 in bottom half 20. As may be seen in Fig. 3, the opposite ends of these pins are adapted to be press fitted into a plurality of bores 142 in top half 18.

Again returning to Fig. 4A, stylet 36 has a reduced diameter shank portion 144 which is dimensioned to be axially slidable in an aperture 47 in front wall 34 and projection 49. An elongated horizontally disposed slot 146 is formed in the proximal end of shank 144. This slot is adapted to receive lever arm 148. Lever arm 148 is pivotally connected at its distal end to shank 144 by means of a pivot pin 150 which passes through bore 152 in the distal end of lever arm 148 and is press fit into bore 154 which is drilled through shank 144. The proximal end of lever arm 148 has a lateral projection 156 at a right angle to the direction of lever arm 148 and a bore 158 therethrough. An elongated actuator pin 160 is fixedly mounted within said bore intermediate its ends by welding or other convenient means.

The upper end portion of actuator pin 160 extends into an arcuate slot 162 in top half 18 and has an actuating lever 164 mounted thereon. A projection 161 depending from the underside of actuating lever 164 travels in groove 162 and is adapted to contact and move pin 160 when the lever is rotated about pivot 163 within aperture 165 in top half 18. Lever 164 is retained within aperture 165 by means of a retainer screw 167 threadedly engaged within an axial bore in pivot 163. The slot 162 is cut to the rear so as to form an axially directed wall 166 for a purpose to be described hereinafter. Material is cut away so as to form an indentation 168 in top half 18. An upward projection 171 facilitates manipulation of lever 164. Shaft 160 is of such a length as to permit a free end thereof to travel within slot 162. A spring 169 around and fixed at one end to pivot 163 and at the other end to a hole 173 in top half 18 biases lever 164 to the position shown in Fig. 1. In the fully retracted position, pin 160 is permitted to reach a position near bottom wall 192 within slot 162 (see Fig. 4A).

The lower portion of actuator pin 160 extends through and is freely pivotable within a bore 170 in lever arm 172. Lever arm 172 is in turn fixed at its opposite end to cylindrical member 174 intermediate its upper and lower ends 176, 178. A slot 180 extends through the length of member 174. This slot is in line with the direction of lever arm 172. A pivot pin 182 passes through slot 180. The lower end of pivot pin 182 is fixed within bore 184 in the bottom of cavity 136. The upper end is adapted to be fixed within bore 186 in cavity 138 in upper half as seen in Fig. 3. Returning to Fig. 4A, a coil spring 188 has a body portion adapted to encompass member 174. One end 192 of coil spring 188 is adapted to bear against and bias actuator pin 160 against wall 166 and towards the bottom 192 of arcuate slot 162. The other end 194 of coil spring 188 has a curved end and contacts a cylindrical projection 196 that extends from the bottom of cavity 136. A pin 198 is press fit within a bore 200 within projection 196. This pin is adapted to fit within a bore 202 in a corresponding cylindrical projection 203 in cavity 138 as seen in Fig. 3. As seen in this figure and in Fig. 4A, an L-shaped latch 204 is mounted within cavity 138 by means of a screw 206 which passes through a bore 208 at the juncture of the two arms 210, 212 of the latch. The latch 204 may conveniently be injection molded of plastic material.

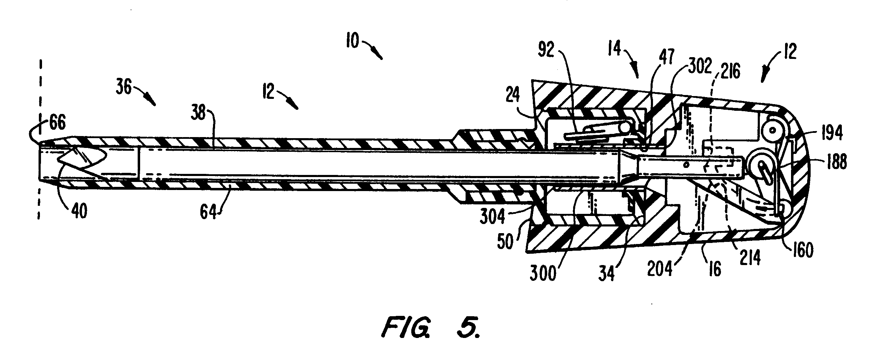

Turning to Fig. 5, there is shown an alternate embodiment of the invention which includes a mechanism for reducing frictional drag on the obturator 38 and thereby on the stylet 36. The drag reduction mechanism comprises a hollow tube 300 which may conveniently be of stainless steel material. The tube is fixedly mounted in an aperture 47 in frustoconical projection 49 on the front wall 34 of head 16 as by press fitting its proximal end 302 therein. The tube is of a length from its proximal end 302 to its distal end 304 so as to hold flap valve mechanism 92 out of contact with the obturator 38 when the two subassemblies 12, 14 are fully engaged as shown. The inner diameter of tube 300 is slightly larger than the outer diameter of obturator 38 so that no frictional drag is imparted to the obturator by the flap valve mechanism 92 when the obturator moves axially incident to operation of the trocar. Tube 300 will eliminate any frictional impedance that would tend to slow the action of spring 188 upon retraction of obturator 38.

The operation of the trocar will now be discussed as follows. Prior to use, the trocar tube assembly 10 will be in the assembled form shown in Fig. 2A. In this form, trocar tube subassembly 12 will have its body 50 nestled within slot 24. Obturator 38 of stylet 36 will be positioned as shown within trocar tube 64. In this initial position or state, tip 40 is recessed within the tube and spaced from the distal end 66. In this manner, the tip is prevented from causing injury during transport. In order to place the trocar in operational condition or form, actuator pin 160 is manually advanced in the arrow direction by moving lever 164 by projection 171 so that projection 161 moves pin 160 as shown in Fig. 4A until it reaches the end of its travel in slot 162. Near the end of its travel, pin 160 will contact rearward-facing transverse edge 214 of latch 204, which projects into slot 162 (see Fig. 3).

Continued movement of pin 160 will move the latch to the left or distal direction as shown in Fig. 2A until it passes axial edge 216. At this moment, the biasing force of latch 204 will return it to its initial position and pin 160 will be held against axial edge 216, as best seen in Fig. 2B. It will be held in this cocked position by the biasing force of coil spring 188 applied through spring end 192.

In this cocked position, the tip 40 projects a distance D₁ from distal end 66 of tube 64. Arm 148 and lever arm 172 will be axially aligned with stylet 36. As shown in this figure, the trocar is manually advanced against the body tissue shown in dotted lines defining the wall of a body cavity. The trocar is inserted through the tissue defining the wall of the body cavity. In practice, this insertion is made through a small incision in the skin that is created for this purpose.

The trocar is gripped firmly in the hand with the rounded rear wall 22 of the head 16 against the palm and the index and middle fingers extending around the front walls 56, 58 of the trocar body subassembly 14 on either side of tube 64. The reactant force of the tissue will cause a slight "float distance" movement of the stylet 36, arm 148 and lever arm 172 in the proximal direction until the position seen in Fig. 2C is achieved. In this position, the distance between the tip and the open end 66 falls to a distance D₂ of about zero. This small amount of excess movement called the "float distance" is made possible by slot 180 in cylindrical member 174. Parenthetically, the "float distance" may be in the range of 1.27 to 4.67 mm (.050 to .1875 inches), with 3.17 mm (.125 inches) as an optimum.

The movement of pivot pin 182 in slot 180 is designed to be greater than the movement required to release latch 204. The slight additional movement of pin 182 within slot 180 allows for slight changes in surgeon advancement rate or variations in tissue density or resistance to not trigger latch 204 and retract the point 40 prior to penetrating the body cavity.

This movement of pin 182 in slot 180 of cylindrical member 174 will now be described in further detail. In this position, pivot pin 182 is located at the distal end of slot 180 and end 194 of coil spring 188 is rotated so as to produce a biasing force in the distal direction. At the same time, actuator pin 160 moves in the proximal direction until it is free of contacting axial edge 216. The lateral biasing force of end 192 of coil spring 188 causes actuator pin 160 to move laterally until it seats against wall 166 and the apex 218 of slot 162. In this position, the final force generated by tip 40 passing through the tissue is resisted by a positive stop formed in top half 18 in the area of contact between pin 160 and apex 218 where wall 166 intersects slot 162. The balance of the force thus generated will be transmitted to bottom half 20 by means of actuator pin 160 contacting apex 220 formed in a raised portion 222 within cavity 136, as best seen in Fig. 4A.

After the tip passes through the tissue and enters the body cavity, the force on the tip will decrease and in fact cease. As soon as this resistant force is less than a threshold value supplied by the biasing force of end 194 of coil spring 188, actuator pin 160 will move in a distal direction under the influence of such biasing force. As seen in Fig. 2D, actuator pin 160 will move distally against edge 214, compressing and moving latch 204 in a distal direction, since the force generated by end 194 of coil spring 188 is greater than that generated by latch 204. As soon as actuator pin 160 moves distally enough to clear contact with wall 166, it will enter slot 162 and move in the arrow direction under the biasing influence of end 192 and spring 188. Actuator pin 160 will move along the arcuate path defined by slot 162 until it is stopped by projection 161 (see Fig. 4A), against which it bears, reaching the slot bottom 192. It will thus have automatically returned to the position shown in Fig. 2A with the tip 40 fully retracted into tube 64. Of course, unlike the Fig. 2A situation, tube 64 will be within the body cavity. In this manner, viscera and other internal tissues are protected from contact with the piercing tip and potential damage that it might cause. At the same time, the surgeon has a visual indication, by the lever 164 (see Fig. 1) being fully retracted, that the tip has completely retracted.

While holding trocar tube subassembly 12 in place, the trocar body subassembly 10 is manually removed in a proximal direction as seen in Fig. 1. In this position, the flap valve mechanism (not shown) will close as visually indicated by the position of actuating lever 120 being in a transverse position. In this operation, once the tip 40 of stylet 36 clears the opening 100 in grommet-like seal 94, spring 118 will bias flapper pad 112 into contact with the grommet-like seal, as best seen in Fig. 4B. In this manner, opening 100 will be closed and sealed. Gas pressure in the body cavity is thus maintained. In this regard, the stopcock 126 will normally be closed during trocar insertion to maintain the gas pressure within the body cavity. If necessary, the stopcock may be used as a conduit for admitting an additional insufflating gas into the cavity.

After the trocar body subassembly 14 has been separated from the trocar tube subassembly 12, surgical instruments may be inserted into the body cavity by way of the trocar tube subassembly to view internal tissues, perform operations, and/or drain body fluids. Actuating lever 120 can be used to manually open valve 102 to facilitate such procedures and also permit removal of specimens, as well as to deflate the body cavity.

It is to be understood that while the invention has been described above in conjunction with the preferred specific embodiments, the description and examples are intended to illustrate and not limit the scope of the invention, which is defined by the scope of the appended claims.

## Claims

1. A trocar assembly comprising:
an elongated trocar obturator (38) having a piercing tip (40) at its distal end;
a trocar body (16), said trocar obturator (38) having its proximal end mounted in said body (16); and
an elongated trocar tube (64) mounted on a trocar tube body (50), said trocar tube body (50) being engageable with said trocar body (16) wherein said obturator (38) is located within said trocar tube (64), said trocar tube (64) having an open distal end, and a proximal end fixed to said tube body (50);
characterized in that said trocar obturator (38) has its proximal end reciprocably mounted in said trocar body (16) and said trocar assembly having means for extending and retracting said obturator (38) relative to said tube (64) from a first position where said tip (40) is recessed from said distal tube end to a second operative position extended from said distal tube end and thence automatically back to said first position, the latter in response to a distal movement of said tip (40) a predetermined distance incident to a change in force against said tip (40) after the tip (40) passes through a cavity wall, said means for extending and retracting including lever means (148) located in a cavity (136, 138) of said trocar body (16) and operatively connected to said obturator (38), spring means (188, 192, 194) located in said cavity (136, 138), said spring means having first spring biasing means (188, 192) for urging said lever means to move said obturator (38) in a proximal direction, and second spring biasing means (188, 194) for urging said lever means to move said obturator (38) in a distal direction, said second spring biasing means (188, 194) effecting said distal movement of said obturator (38) and tip (40) responsive to said change in force acting against said tip (40) and the first spring biasing means (188, 192) being then effective to retract said obturator (38) to said first position.

2. The trocar assembly of claim 1, characterized in that said change in force is a decrease in force and said predetermined distance is in the range of about 1.27 to about 4.67 mm (.050 to .1875 inches).

3. The trocar assembly of claim 2, characterized in that said predetermined distance is about 3.17 mm (.125 inches).

4. The trocar assembly of claim 1, characterized in that said means for extending and retracting comprises manually operable means (164) operatively connected to said obturator (38).

5. The trocar assembly of claim 1, characterized in that said means for extending and retracting further includes means (204) for holding said obturator (138) in said second position until said tip (40) moves in a distal direction a predetermined distance incident to the change in force on the tip (40) after it has passed through a cavity wall.

6. The trocar assembly of claim 1, characterized in that said lever means comprises a pair of lever arms (148, 172) pivotally connected to each other by means of an actuator pin (160), means pivotally connecting a distal end of one (148) of said lever arms to said obturator (38), the proximal end of the other (172) of said lever arms having a member (174) fixed thereto including a slot (180) therein, and a pivot pin (182) defining a pair of ends within said slot (180), said ends of said pivot pin (182) being fixed to said trocar body (16).

7. The trocar assembly of claim 6, characterized in that said means for extending and retracting further comprises an arcuate slot (162) in said trocar body (16), said actuator pin (160) being of a length to travel within said arcuate slot (162) whereby said obturator (38) is retracted by said first spring biasing means (188, 192).

8. The trocar assembly of claim 7, characterized in that said means for extending and retracting further comprises an axially-directed slot in said trocar body (16) communicating with said arcuate slot (162), and further including latch means (204) having a force loss than that of said second spring biasing means (188, 194), and manually actuatable means (164) for moving said actuator pin (160) in said arcuate slot (162) against the biasing force of said first spring biasing means (188, 192) so as to deflect said latch means (204) until said actuator pin (160) reaches said axially-directed slot wherein said latch means (204) restrains said actuator pin (160) from moving back along said arcuate slot (162) until the proximal force generated by moving said tip (40) through a body wall causes said actuator pin (160) to enter said axially-directed slot whereby said second spring biasing means (188, 194) having a force greater than that of said latch means (204) moves said latch means (204) in a distal direction whereby said actuator pin (160) again enters said arcuate slot (162) and is moved therein by the first spring biasing means (188, 192) thereby retracting said obturator (38).

9. The trocar assembly of claim 8, characterized in that said axially-directed slot includes an apex (218) at its proximal end and a laterally-directed side wall (166), said first spring biasing means (188, 192) causing said actuator pin (160) to be biased against said side wall (166).

10. The trocar assembly of claim 6, characterized in that said member (174) is generally cylindrical in shape and said second spring biasing means (188, 194) is a coil spring around said member (174).

11. The trocar assembly of claim 1, characterized in that said trocar tube body (50) defines a cavity (90) therein, said trocar assembly including valve means (92) in said cavity (90) for selectively closing off said proximal end of said tube (64).

12. The trocar assembly of claim 11, characterized in that said valve means (92) comprises a generally circular seal (94) having an aperture (100) therethrough adapted for passage of surgical instruments.

13. The trocar assembly of claim 12, characterized in that said valve means (92) further comprises a valve (102) and means pivotally mounting said valve (102) so that it may selectively close off said aperture (100).

14. The trocar assembly of claim 13, characterized in that said means pivotally mounting said valve (102) is an elongated shaft (104), said valve (102) being mounted to said shaft (104) along one edge thereof, and further including means pivotally mounting said shaft (104) in said tube body (50).

15. The trocar assembly of claim 14, characterized in that said valve means (92) further comprises spring means (118) biasing said valve (102) to the closed position obturating said aperture (100).

16. The trocar assembly of claim 14, characterized in that said valve means (92) further comprises a lever (120) fixed to said shaft (104) so that the valve (92) may be manually pivoted so as to open said aperture (100).

17. The trocar assembly of claim 12, characterized in that said obturator (38) is generally cylindrical defining a first diameter and having a reduced diameter shank portion (144) at its proximal end, said aperture (100) being circular and having a diameter less than that of said first diameter and greater than that of said reduced diameter so that there is no frictional drag on said obturator (38) from said seal (94) when said obturator (38) is in the second position.

18. The trocar assembly of claim 13, characterized by including means for holding said valve (102) away from said obturator (38) when said trocar body (16) and tube body (50) are engaged so as to prevent frictional drag on said obturator (38) from said valve (102).

19. The trocar assembly of claim 18, characterized in that said means for holding said valve (102) away from said obturator (38) comprises a tubular member (300) on said trocar body (16) located so as to surround and be intermediate said valve (102) and said obturator (38).

20. The trocar assembly of claim 1, characterized in that said trocar body (16) includes a slot (24) in a forward wall thereof and wherein said tube body (50) is dimensioned to closely fit within said slot (24) in said forward wall.

21. The trocar assembly of claim 13, characterized by means to reduce friction caused by said valve (102) contacting said obturator (38), said friction reducing means comprising a proximal shank portion (144) of said obturator (38), being of a reduced diameter compared with the diameter of the obturator (38) in advance of said shank portion (144).

22. The trocar assembly of claim 8, characterized by including a valve (128) in said tube body (50) for selectively intercommunicating said cavity (90) in said tube body (50) with the exterior of said tube body (50) whereby insufflating gas may be admitted to and contained within said tube body (50) and body cavity (90).

## Patentansprüche

1. Trokarvorrichtung mit:
einem langgestreckten Trokarobturator (38), der eine Stechspitze (40) an seinem distalen Ende hat;
einem Trokarkörper (16), wobei der Trokarobturator (38) mit seinem proximalen Ende in dem Körper (16) befestigt ist; und
einem langgestreckten Trokarrohr (64), das an einem Trokarrohrkörper (50) befestigt ist, wobei der Trokarrohrkörper (50) mit dem Trokarkörper (16) in Eingriff bringbar ist, wobei der Obturator (38) in dem Trokarrohr (64) angeordnet ist und wobei das Trokarrohr (64) ein offenes, distales Ende und ein an dem Rohrkörper (50) befestigtes proximales Ende hat;
dadurch gekennzeichnet, daß der Trokarobturator (38) mit seinem proximalen Ende in dem Trokarrohr (16) hin- und herbewegbar gelagert ist und die Trokarvorrichtung eine Einrichtung hat zum Ausfahren und Zurückziehen des Obturators (38) relativ zu dem Rohr (64) aus einer ersten Position, in welcher die Spitze (40) von dem distalen Rohrende zurückgezogen ist, in eine zweite, wirksame Position, in der sie aus dem distalen Rohrende ausgefahren ist, und von dieser aus automatisch zurück in die erste Position, letzteres aufgrund einer distalen Bewegung der Spitze (40) um eine vorbestimmte Strecke bei einer Änderung der auf die Spitze (40) einwirkenden Kraft, nachdem die Spitze (40) durch eine Körperhöhlenwand hindurchgegangen ist, wobei die Einrichtung zum Ausfahren und Zurückziehen eine Hebeleinrichtung (148) aufweist, die in einem Hohlraum (136, 138) des Trokarkörpers (16) angeordnet ist und mit dem Obturator (38) in Wirkverbindung steht, eine Federeinrichtung (188, 192, 194), die in dem Hohlraum (136, 138) angeordnet ist, wobei die Federeinrichtung eine erste Federvorspanneinrichtung (188, 192) hat zum Drängen der Hebeleinrichtung, den Obturator (38) in einer proximalen Richtung zu bewegen, und eine zweite Federvorspanneinrichtung (188, 194) zum Drängen der Hebeleinrichtung, den Obturator (38) in einer distalen Richtung zu bewegen, wobei die zweite Federvorspanneinrichtung (188, 194) die distale Bewegung des Obturators (38) und der Spitze (40) auf Grund der Änderung der Kraft bewirkt, die auf die Spitze (40) einwirkt, und die erste Federvorspanneinrichtung (188, 192) bewirkt, daß der Obturator (38) in die erste Position zurückgezogen wird.

2. Trokarvorrichtung nach Anspruch 1, dadurch gekennzeichnet, daß die Änderung der Kraft eine Verringerung der Kraft ist und daß die vorbestimmte Strecke in dem Bereich von etwa von 1,27 bis etwa 4,67 mm (0.050 bis 0.1875 Zoll) liegt.

3. Trokarvorrichtung nach Anspruch 2, dadurch gekennzeichnet, daß die vorbestimmte Strecke etwa 3,17 mm (0.125 Zoll) beträgt.

4. Trokarvorrichtung nach Anspruch 1, dadurch gekennzeichnet, daß die Einrichtung zum Ausfahren und Zurückziehen eine manuell betätigbare Einrichtung (164) aufweist, die mit dem Obturator (38) in Wirkverbindung steht.

5. Trokarvorrichtung nach Anspruch 1, dadurch gekennzeichnet, daß die Einrichtung zum Ausfahren und Zurückziehen weiter eine Einrichtung (204) aufweist zum Halten des Obturators (138) in der zweiten Position, bis sich die Spitze (40) in einer distalen Richtung um eine vorbestimmte Strecke bei der Änderung der auf die Spitze (40) einwirkenden Kraft, nachdem diese durch eine Körperhöhlenwand hindurchgegangen ist, bewegt hat.

6. Trokarvorrichtung nach Anspruch 1, dadurch gekennzeichnet, daß die Hebeleinrichtung ein Paar Hebelarme (148, 172) aufweist, die mittels eines Betätigungsstifts (160) drehbar miteinander verbunden sind, eine Einrichtung zum drehbaren Verbinden eines distalen Endes von einem (148) der Hebelarme mit dem Obturator (38), wobei das proximale Ende des anderen Hebelarms (172) ein an ihm befestigtes Teil (174) hat, in welchem ein Schlitz (180) vorgesehen ist, und einen Drehstift (182) mit zwei Enden in dem Schlitz (180), wobei aie Enden des Drehstifts (182) an dem Trokarkörper (16) befestigt sind.

7. Trokarvorrichtung nach Anspruch 6, dadurch gekennzeichnet, daß die Einrichtung zum Ausfahren und Zurückziehen weiter einen bogenförmigen Schlitz (162) in dem Trokarkörper (16) aufweist und daß der Betätigungsstift (160) so lang ist, daß er sich in dem bogenförmigen Schlitz (162) bewegt, wodurch der Obturator (38) durch die erste Federvorspanneinrichtung (188, 192) zurückgezogen wird.

8. Trokarvorrichtung nach Anspruch 7, dadurch gekennzeichnet, daß die Einrichtung zum Ausfahren und Zurückziehen weiter einen axial gerichteten Schlitz in dem Trokarkörper (16) aufweist, der mit dem bogenförmigen Schlitz (162) in Verbindung steht, und weiter eine Verriegelungeinrichtung (204), die eine Kraft hat, welche kleiner ist als die der zweiten Federvorspanneinrichtung (188, 194), und eine manuell betätigbare Einrichtung (164) zum Bewegen des Betätigungsstifts (160) in dem bogenförmigen Schlitz (162) entgegen der Vorspannkraft der ersten Federvorspanneinrichtung (188, 192), um so die Verriegelungseinrichtung (204) auszulenken, bis der Betätigungsstift (160) den axial gerichteten Schlitz erreicht, wobei die Verriegelungseinrichtung (204) den Betätigungsstift (160) daran hindert, sich längs des bogenförmigen Schlitzes (162) zurückzubewegen, bis die proximale Kraft, welche durch das Hindurchbewegen der Spitze (40) durch eine Körperwand erzeugt wird, bewirkt, daß der Betätigungsstift (160) in den axial gerichteten Schlitz eindringt, wodurch die zweite Federvorspanneinrichtung (188, 194), deren Kraft größer ist als die der Verriegelungseinrichtung (204), die Verriegelungseinrichtung (204) in einer distalen Richtung bewegt, wodurch der Betätigungsstift (160) wieder in den bogenförmigen Schlitz (162) eintritt und darin durch die erste Federvorspanneinrichtung (188, 192) bewegt wird, wodurch der Obturator (38) zurückgezogen wird.

9. Trokarvorrichtung nach Anspruch 8, dadurch gekennzeichnet, daß der axial gerichtete Schlitz einen Scheitel (218) an seinem proximalen Ende und eine lateral gerichtete Seitenwand (166) aufweist und daß die erste Federvorspanneinrichtung (188, 192) bewirkt, daß der Betätigungsstift (160) gegen die Seitenwand (166) vorgespannt wird.

10. Trokarvorrichtung nach Anspruch 6, dadurch gekennzeichnet, daß das Teil (174) eine insgesamt zylindrische Form hat und daß die zweite Federvorspanneinrichtung (188, 194) eine Schraubenfeder um das Teil (174) ist.

11. Trokarvorrichtung nach Anspruch 1, dadurch gekennzeichnet, daß der Trokarrohrkörper (50) einen Hohlraum (90) aufweist und daß die Trokarvorrichtung eine Ventileinrichtung (92) in dem Hohlraum (90) zum wahlweisen Absperren des proximalen Endes des Rohres (64) aufweist.

12. Trokarvorrichtung nach Anspruch 11, dadurch gekennzeichnet, daß die Ventileinrichtung (92) eine insgesamt kreisförmige Dichtung (94) aufweist, die eine durchgehende Öffnung (100) zum Hindurchführen von chirurgischen Instrumenten hat.

13. Trokarvorrichtung nach Anspruch 12, dadurch gekennzeichnet, daß die Ventileinrichtung (92) weiter ein Verschlußstück (102) und eine Einrichtung zum schwenkbaren Befestigen des Verschlußstückes (102) aufweist, so daß es die Öffnung (100) wahlweise verschließen kann.

14. Trokarvorrichtung nach Anspruch 13, dadurch gekennzeichnet, daß die Einrichtung zum schwenkbaren Befestigen des Verschlußstücks (102) eine langgestreckte Welle (104) ist, wobei das Verschlußstück (102) an der Welle (104) längs eines Randes derselben befestigt ist, und weiter eine Einrichtung aufweist zum schwenkbaren Lagern der Welle (104) in dem Rohrkörper (50).

15. Trokarvorrichtung nach Anspruch 14, dadurch gekennzeichnet, daß die Ventileinrichtung (92) weiter eine Federeinrichtung (118) aufweist, die das Verschlußstück (102) in die geschlossene Stellung, in der es die Öffnung (100) verschließt, vorspannt.

16. Trokarvorrichtung nach Anspruch 14, dadurch gekennzeichnet, daß die Ventileinrichtung (92) weiter einen Hebel (120) aufweist, der an der Welle (104) befestigt ist, so daß das Verschlußstück (92) manuell geschwenkt werden kann, um so die Öffnung (100) zu verschließen.

17. Trokarvorrichtung nach Anspruch 12, dadurch gekennzeichnet, daß der Obturator (38) insgesamt zylindrisch ist und einen ersten Durchmesser aufweist sowie einen Schaftteil (144) reduzierten Durchmessers an seinem proximalen Ende hat, wobei die Öffnung (100) kreisförmig ist und einen Durchmesser hat, der kleiner als der erste Durchmesser und größer als der reduzierte Durchmesser ist, so daß es keinen Reibungswiderstand an dem Obturator (38) von der Dichtung (94) her gibt, wenn der Obturator (38) in der zweiten Position ist.

18. Trokarvorrichtung nach Anspruch 13, gekennzeichnet durch eine Einrichtung zum Weghalten des Verschlußstücks (102) von dem Obturator (38), wenn der Trokarkörper (16) und der Rohrkörper (50) in Eingriff sind, um so einen Reibungswiderstand an dem Obturator (38) von dem Verschlußstück (102) her zu verhindern.

19. Trokarvorrichtung nach Anspruch 18, dadurch gekennzeichnet, daß die Einrichtung zum Weghalten des Ventilverschlußstücks (102) von dem Obturator (38) ein rohrförmiges Teil (300) an dem Trokarkörper (16) aufweist, das so angeordnet ist, daß es das Verschlußstück (102) und den Obturator (38) umgibt und zwischen denselben angeordnet ist.

20. Trokarvorrichtung nach Anspruch 1, dadurch gekennzeichnet, daß der Trokarkörper (16) einen Schlitz (24) in einer vorderen Wand aufweist und daß der Rohrkörper (50) so bemessen ist, daß er eng in den Schlitz (24) in der vorderen Wand paßt.

21. Trokarvorrichtung nach Anspruch 13, gekennzeichnet durch eine Einrichtung zum Reduzieren der Reibung, die durch das Verschlußstück (102) verursacht wird, welches den Obturator (38) berührt, wobei die Reibungsreduziereinrichtung einen proximalen Schaftteil (144) des Obturators (38) aufweist, der einen im Vergleich zu dem Durchmesser des Obturators (38) vorderhalb des Schaftteils (144) reduzierten Durchmesser hat.

22. Trokarvorrichtung nach Anspruch 8, gekennzeichnet durch ein Ventil (128) in dem Rohrkörper (50) zum wahlweisen Miteinanderverbinden des Hohlraums (90) in dem Rohrkörper (50) mit der äußeren Umgebung des Rohrkörpers (50), wodurch Insuffliergas in den Rohrkörper (50) und in die Körperhöhle (90) eingeleitet und darin eingeschlossen werden kann.

## Revendications

1. Un ensemble de trocart comprenant un obturateur de trocart (38) allongé, ayant une pointe de perçage (40) à son extrémité distale, un corps de trocart (16) dans lequel est montée l'extrémité proximale de l'obturateur de trocart (38), et un tube de trocart allongé (64) monté sur un corps de tube de trocart (50), ce corps de tube de trocart (50) pouvant être mis en contact avec le corps de trocart (16) de telle façon que l'obturateur (38) soit logé à l'intérieur du tube de trocart (64), ce tube de trocart (64) ayant une extrémité distale ouverte et une extrémité proximale fixée au corps de tube (50), caractérisé en ce que l'obturateur de trocart (38) a son extrémité proximale montée d'une manière alternative dans le corps de trocart (16) et l'ensemble de trocart comporte un moyen pour étendre et rétracter l'obturateur (38) par rapport au tube (64), à partir d'une première position dans laquelle la pointe (40) est escamotée à partir de l'extrémité distale du tube, jusqu'à une seconde position opérationnelle dans laquelle la pointe est étendue à partir de l'extrémité distale du tube et est déplacée automatiquement en sens inverse pour retourner à la première position, cette opération de retour s'effectuant en réponse à un mouvement distal de la pointe (40) sur une distance prédéterminée à la suite d'un changement de la force s'exerçant contre la pointe (40), après que cette pointe (40) a passé à travers une paroi d'une cavité, le moyen pour étendre et rétracter l'obturateur comportant un moyen à levier (148) logé dans une cavité (136,138) du corps (16) du trocart et connecté opérationnellement à l'obturateur (38), des moyens à ressort (188,192,194) logés dans la cavité (136,138), ces moyens à ressort comportant un premier moyen de sollicitation à ressort (188,192) pour solliciter le moyen à levier de manière à déplacer l'obturateur (38) dans une direction proximale, et un second moyen de sollicitation à ressort (188,194) pour solliciter le moyen à levier de manière à déplacer l'obturateur (38) dans une direction distale, le second moyen de sollicitation à ressort (188,194) provoquant le mouvement distal de l'obturateur (38) et de la pointe (40) en réponse au changement de la force agissant contre la pointe (40) et le premier moyen de sollicitation à ressort (188,192) intervenant ensuite pour rétracter l'obturateur (38) dans sa première position.

2. L'ensemble de trocart de la revendication 1 caractérisé en ce que la variation de la force est une diminution de cette force et la distance prédéterminée est comprise dans la plage allant d'environ 1,27 à environ 4,67 mm (0,050 à 0,1875 pouce).

3. L'ensemble de trocart de la revendication 2 caractérisé en ce que la distance prédéterminée est d'environ 3,17 mm (0,125 pouce).

4. L'ensemble de trocart de la revendication 3 caractérisé en ce que le moyen pour étendre et rétracter l'obturateur comprend un moyen (164) actionnable manuellement et connecté opérationnellement à l'obturateur (38).

5. L'ensemble de trocart de la revendication 1 caractérisé en ce que le moyen pour étendre et rétracter l'obturateur comporte en outre un moyen (204) pour maintenir l'obturateur (138) dans sa seconde position jusqu'à ce que la pointe (40) se soit déplacée, dans une direction distale, d'une distance prédéterminée à la suite d'un changement de la force s'exerçant sur la pointe (40) après que celle-ci a passé à travers la paroi d'une cavité.

6. L'ensemble de trocart de la revendication 1 caractérisé en ce que le moyen à levier comprend une paire de bras de levier (148,172) reliés à pivotement l'un à l'autre au moyen d'un axe d'actionneur (160), un moyen connectant à pivotement une extrémité distale de l'un (148) des bras de levier à l'obturateur (38), l'extrémité proximale de l'autre (172) des bras de levier ayant un organe (174) qui lui est fixé et qui comporte une fente (180) à l'intérieur, et un axe de pivotement (182) définissant une paire d'extrémités dans cette fente, les extrémités de cet axe de pivotement (182) étant fixées au corps de trocart (16).

7. L'ensemble de trocart de la revendication 6 caractérisé en ce que le moyen pour étendre et rétracter l'obturateur comprend en outre une fente arquée (162) dans le corps de trocart (16), l'axe d'actionneur (160) ayant une longueur de course dans cette fente arquée (162) si bien que l'obturateur (38) est rétracté par le premier moyen de sollicitation à ressort (188,192).

8. L'ensemble de trocart de la revendication 7 caractérisé en ce que le moyen pour étendre et rétracter l'obturateur comprend en outre une fente dirigée axialement dans le corps de trocart (16), communiquant avec la fente arquée (162), et il comporte en outre un moyen de verrouillage (204) ayant une force inférieure à celle du second moyen de sollicitation à ressort (188,194), et un moyen (164) actionnable manuellement pour déplacer le doigt d'actionneur (160) dans la fente arquée (162), à l'encontre de la force de rappel du premier moyen de sollicitation à ressort (188,192), de manière à fléchir le moyen de verrouillage (204) jusqu'à ce que le doigt d'actionneur (160) atteigne la fente dirigée axialement, le moyen de verrouillage (204) empêchant le doigt d'actionneur (160) de se déplacer en arrière le long de la fente arquée (162) jusqu'à ce que la force proximale produite par le déplacement de la pointe (40) à travers une paroi du corps amène le doigt d'actionneur (160) à pénétrer dans la fente dirigée axialement de telle façon que le second moyen de sollicitation à ressort (188,194), exerçant une force supérieure à celle du moyen de verrouillage (204), déplace ce moyen de verrouillage (204) dans une direction distale si bien que le doigt d'actionneur (160) pénètre de nouveau dans la fente arquée (162) et est déplacé dans celle-ci par le premier moyen de sollicitation à ressort (188,192), en rétractant ainsi l'obturateur (38).

9. L'ensemble de trocart de la revendication 8 caractérisé en ce que la fente dirigée axialement comporte un sommet (218) à son extrémité proximale et une paroi de côté dirigée latéralement (166) et le premier moyen de sollicitation à ressort (188,192) amène le doigt d'actionneur (160) à être pressé contre cette paroi de côté (166).

10. L'ensemble de trocart de la revendication 6 caractérisé en ce que l'organe (174) a une forme généralement cylindrique et le second moyen de sollicitation à ressort (188,194) est un ressort hélicoïdal entourant cet organe (174).

11. L'ensemble de trocart de la revendication 1 caractérisé en ce que le corps de tube de trocart (50) définit à l'intérieur une cavité (90), cet ensemble de trocart comportant un moyen à clapet (92) à l'intérieur de la cavité (90) pour obturer sélectivement l'extrémité proximale du tube (64).

12. L'ensemble de trocart de la revendication 11 caractérisé en ce que le moyen à clapet (92) comprend un joint d'étanchéité (94) généralement circulaire ayant une ouverture (100) à travers lui laquelle est adaptée pour le passage d'instruments chirurgicaux.

13. L'ensemble de trocart de la revendication 12 caractérisé en ce que le moyen à clapet (92) comprend en outre un clapet (102) et un moyen pour monter à pivotement ce clapet (102) de telle façon qu'il puisse obturer sélectivement l'ouverture (100).

14. L'ensemble de trocart de la revendication 13 caractérisé en ce que le moyen assurant le montage à pivotement du clapet (102) est un arbre allongé (104), le clapet (102) étant monté sur cet arbre (104), le long de l'un de ses côtés, et comportant en outre un moyen pour monter à pivotement l'arbre (104) dans le corps de tube (50).

15. L'ensemble de trocart de la revendication 14 caractérisé en ce que le moyen à clapet (92) comprend en outre un moyen élastique (118) sollicitant le clapet (102) vers la position fermée obturant l'ouverture (100).

16. L'ensemble de trocart de la revendication 14 caractérisé en ce que le moyen à clapet (92) comprend en outre un levier (120) fixé à l'arbre (104) de telle façon que le clapet (92) puisse pivoter manuellement pour ouvrir l'ouverture (100).

17. L'ensemble de trocart de la revendication 12 caractérisé en ce que l'obturateur (38) est généralement cylindrique en définissant un premier diamètre et ayant une portion de fût (144) de diamètre réduit à son extrémité proximale, l'ouverture (100) étant circulaire et ayant un diamètre inférieur à celui du premier diamètre et supérieur à celui du diamètre réduit si bien qu'il n'y a pas de résistance de frottement s'exerçant sur l'obturateur (38) de la part du joint d'étanchéité (94) lorsque l'obturateur (38) se trouve dans la seconde position.

18. L'ensemble de trocart de la revendication 13 caractérisé en ce qu'il comporte en outre un moyen pour maintenir le clapet (102) à distance de l'obturateur (38) lorsque le corps de trocart (16) et le corps de tube (50) sont engagés l'un dans l'autre, de manière à éviter une résistance de frottement sur l'obturateur (38) de la part du clapet (102).

19. L'ensemble de trocart de la revendication 18 caractérisé en ce que le moyen pour maintenir le clapet (102) à distance de l'obturateur (38) comprend un organe tubulaire (300) sur le corps de trocart (16), disposé de manière à entourer et à se trouver entre le clapet (102) et l'obturateur (38).

20. L'ensemble de trocart de la revendication 1 caractérisé en ce que le corps de trocart (16) comporte une fente (24) dans une paroi antérieure de ce corps et le corps de tube (50) est dimensionné de façon à s'emboîter étroitement dans cette fente (24) prévue dans la paroi antérieure.

21. L'ensemble de trocart de la revendication 13 caractérisé en ce qu'il comporte un moyen pour réduire le frottement provoqué par le clapet (102) venant en contact avec l'obturateur (38), ce moyen réduisant le frottement comprenant une portion de fût proximale (144) de l'obturateur (38) qui a un diamètre réduit comparativement au diamètre de l'obturateur (38) en avant de cette portion de fût (144).

22. L'ensemble de trocart de la revendication 8 caractérisé en ce qu'il comporte un clapet (128) dans le corps de tube (50) pour mettre sélectivement en communication la cavité (90) dans le corps de tube (50) avec l'extérieur de ce corps de tube (50), de telle façon qu'un gaz d'insufflation puisse être admis dans le corps de tube (50) et dans la cavité (90) du corps et être contenu dans ceux-ci.
